# EUROPEAN PATENT APPLICATION

(11) **EP 4 818 333 A1**
(43) Date of publication of application: **07.10.2026**
(21) Application number: 25167316.6
(22) Date of filing: 31.03.2025
(51) Int. Cl.: C12Q 1/6886

(54) **METHODS, BIOMARKERS, AND KITS FOR DETECTION OF MULTIPLE MYELOMA**

(71) Applicant: Universität Heidelberg, 69117 Heidelberg (DE); Universitätsklinikum Heidelberg, 69120 Heidelberg (DE)
(72) Inventor: HUNDEMER, Michael, 69120 Heidelberg (DE); AWWAD, Mohamed, 69120 Heidelberg (DE); TON, Gigi Nu Hoang Quy, 69120 Heidelberg (DE); ROTH, Kevin, 69120 Heidelberg (DE); SCHMITT, Kerstin, 69120 Heidelberg (DE)
(74) Representative: Cohausz & Florack

(57) **Abstract**

The present invention relates to an *in vitro* method for detecting multiple myeloma in a subject. The invention further provides the use of chemokine receptor CXCR3 (CXCR3) as a biomarker for diagnosing, prognosing, and/or treatment monitoring of multiple myeloma in a subject. The invention further provides a kit for the detection of multiple myeloma in a subject.

## Description

### FIELD OF THE INVENTION

The present invention relates to methods for detecting multiple myeloma in a subject. The invention further relates to biomarkers for diagnosing, prognosing, and/or treatment monitoring of multiple myeloma. The invention further relates to a kit for the detection of multiple myeloma in a subject.

### BACKGROUND OF THE INVENTION

Multiple myeloma (MM) is a hematologic malignancy characterized by the clonal proliferation of plasma cells within the bone marrow. Plasma cells, derived from B cells, play a crucial role in the immune system by producing immunoglobulins (antibodies). In multiple myeloma, these plasma cells become neoplastic, leading to the overproduction of monoclonal immunoglobulins or light chains, which can result in various clinical manifestations, including bone pain, anemia, renal dysfunction, and hypercalcemia.

Although effective therapies for the treatment of MM exist, it is of paramount importance for the overall efficacy and success of the treatment to constantly monitor the response of MM patient to the therapy throughout the treatment, as well as to monitor the patient's MM status post-treatment once remission has been achieved.

In MM, assessing the patient's minimal residual disease (MRD) status has become crucial for evaluating treatment efficacy and predicting patient outcomes.

MRD refers to the small number of cancer cells that persist in a patient's body during or after anti-cancer treatment when the patient is or appears to be in remission. These cancer cells cannot be detected by conventional diagnostic methods used to determine the treatment success of the anti-cancer treatment but can be detected by highly sensitive detection methods such as polymerase chain reaction (PCR), next-generation flow cytometry (NGF) or next-generation sequencing (NGS). These cancer cells have the potential to cause relapse in the patient.

Numerous studies have demonstrated that achieving a negative MRD status is associated with improved progression-free survival (PFS) and overall survival (OS) in MM patients. A meta-analysis encompassing various patient populations, including those with newly diagnosed and relapsed MM, revealed that an MRD-negative status correlated with superior PFS and OS compared to an MRD-positive status. This correlation underscores the prognostic value of MRD assessment in guiding treatment decisions and predicting long-term patient outcomes. The prognostic significance of MRD negativity has led to its consideration as a surrogate endpoint in clinical trials. In April 2024, the U.S. Food and Drug Administration's Oncologic Drugs Advisory Committee (ODAC) unanimously voted in favor of using MRD as an early endpoint for accelerated approval in MM clinical trials. This decision facilitates the expedited approval of new therapies by providing an earlier indication of clinical benefit. Similarly, the European Medicines Agency has issued reflection papers discussing the potential of MRD as a clinical endpoint in MM studies, indicating a global recognition of its importance.

Advancements in MM therapies have led to deeper and more sustained patient responses, necessitating sensitive techniques to detect MRD. MRD assessment in MM is typically performed using NGF or NGS on bone marrow samples, with sensitivities reaching 10⁻⁵ to 10⁻⁶. These methods enable the detection of one malignant cell among 100,000 to 1,000,000 normal cells, providing a more precise evaluation of the disease status than conventional diagnostic methods. Despite the high sensitivity of the NGF and NGS methods, investigating a patient's MRD status on bone marrow samples is associated with significant limitations. Retrieving bone marrow aspirates is a painful and invasive process causing patient discomfort and limiting the frequency of MRD testing, and the variability in the bone marrow aspirates, e.g., hemodilution, can reduce the accuracy and the reproducibility of the MRD detection. Furthermore, in MM, the malignant plasma cells can be heterogeneously distributed in the bone marrow increasing the risk of false-negative results if the sampled region does not contain residual disease.

Therefore, there is an unmet need for new and improved methods to detect MM in a patient with high sensitivity, specificity, precision, and accuracy. In particular, for new and improved methods that allow for minimally invasive and systemic detection of MM in patients, e.g., the MRD status of a MM patient, with high sensitivity, specificity, precision, and accuracy. This would allow determining the MRD status of MM patients more frequently and allow guiding of treatment decisions based on the patient's MRD status.

### SUMMARY OF THE INVENTION

Against the aforementioned background, it is therefore an object of the present invention to provide improved means and methods for the detection of MM in a subject with high sensitivity, specificity, precision, and accuracy.

It is also an object of the present invention to provide improved means and methods for the minimally invasive and systemic detection of MM in a subject with high sensitivity, specificity, precision, and accuracy.

It is also an object of the present invention to provide improved means and methods for the detection of MRD of MM in a subject with high sensitivity, specificity, precision, and accuracy.

It is also an object of the present invention to provide improved means and methods for the minimally invasive and systemic detection of MRD of MM in a subject with high sensitivity, specificity, precision, and accuracy.

It is a further object of the present invention to provide a biomarker for the detection of MM in a subject with high sensitivity, specificity, precision, and accuracy, in particular for the minimally invasive and systemic detection of MM.

It is also an object of the present invention to provide a biomarker for the detection of MRD of MM in a subject with high sensitivity, specificity, precision, and accuracy, in particular for the minimally invasive and systemic detection of MRD of MM.

These objects are achieved by the invention set forth in the claims and embodiments explained in more detail below.

The invention concerns an *in vitro* method for detecting multiple myeloma (MM) in a subject, wherein the method comprises:
a. obtaining a biological sample from the subject, and
b. determining the presence of the biomarker chemokine receptor CXCR3 (CXCR3) in the biological sample, wherein the presence of CXCR3 in the biological sample is indicative of MM in the subject.

The invention further provides the use of chemokine receptor CXCR3 (CXCR3) as a biomarker for diagnosing, prognosing, and/or treatment monitoring of multiple myeloma (MM) in a subject.

Furthermore, the invention concerns the use of an *in vitro* method for detecting multiple myeloma (MM) in a subject as described herein for diagnosing, prognosing, and/or treatment monitoring of multiple myeloma in a subject.

The invention also provides a kit for the detection of multiple myeloma in a subject, wherein the kit comprises:
- a first primer pair which is capable of amplifying a target polynucleotide sequence corresponding to the biomarker chemokine receptor CXCR3, and
- a first DNA probe which is capable of binding to the target polynucleotide sequence.

The detection of MM in a subject or patient, particularly the subject's MRD status, is crucial for determining MM treatment efficiency and predicting long term treatment success. A key challenge of MRD detection in subjects afflicted with MM is to combine the high sensitivity, specificity, precision, and accuracy of a detection method with minimally invasive and systemic detection.

In the research underlying the invention, it was surprisingly found that MRD could be detected in the peripheral blood of MM subjects by detecting splice variant A of the biomarker CXCR3 using a droplet digital PCR (ddPCR) assay (cf. example 1). The CXCR3-A ddPCR assay correlated with the results of the established NGF analysis of bone marrow aspirates obtained from the same subjects and was able to determine MRD in the subjects with high sensitivity, specificity, precision, and accuracy.

Advantageous embodiments of the invention are indicated in the dependent claims and in the following.

### DETAILED DESCRIPTION

Although certain embodiments of the present invention are described in detail below, it is to be understood that this invention is not limited to the particular embodiments, methodologies, protocols and reagents described herein as these may vary within the scope set by the claims. It is also to be understood that terminology used herein is for the purpose of describing particular embodiments only, and is not intended to limit the scope of the present invention which is defined by the appended claims. Unless defined otherwise, all technical and scientific terms used herein have the same meanings as commonly understood by a skilled person in the field of gene therapy, biochemistry, genetics, and molecular biology.

In the following description, certain elements of the present invention will be described. These elements may be discussed with specific embodiments, however, it should be understood that they may be combined in any manner and in any number to create additional embodiments. The variously described examples, features and particular embodiments should not be construed to limit the present invention to only the explicitly described embodiments or to the explicitly described combination of features. This description should be understood to disclose and encompass embodiments which combine the explicitly described embodiments with any number of the disclosed and/or preferred elements. Furthermore, any permutations and combinations of all described elements in this application should be considered disclosed by this description unless the context indicates otherwise.

### Definitions

Unless indicated otherwise, the technical and scientific terms used in this specification have the meaning commonly understood by a person skilled in the art in the fields of biotechnology and medical diagnostics. Standard techniques and methodologies referenced herein, including but not limited to molecular biology, biochemistry, cell culture, and analytical methods, are well known and routinely used by those skilled in the art. Any definitions provided throughout the present specification are intended as an addition to the established meanings known in the art and serve only to clarify the most relevant terms in the context of the present disclosure.

The term "CXCR3" as used herein refers to the chemokine receptor CXCR3. In some embodiments, CXCR3 is used as a biomarker. In some embodiments, CXCR3 is used as a biomarker for the detection of MM, particularly for the detection of MRD of MM.

The terms "indicative of multiple myeloma" or "indicative of MM" are used interchangeably herein and refer to the correlation between the detection of a biomarker in a biological sample and the presence of MM in the subject from which the biological sample is obtained. In particular, the term "the presence of CXCR3 in the biological sample is indicative of MM in the subject" means that the detection of CXCR3 in the biological sample, i.e., the presence of CXCR3 in the biological sample, correlates with and/or evidences that the subject has MM.

The term "biological sample" as used herein refers to biological material that is obtained from a subject. For example, a biological sample can be or can be derived from the blood, bone marrow, tissue, urine, and/or saliva of a subject.

The terms "minimal residual disease", "measurable residual disease", or "MRD" are used interchangeably herein and refer to the small number of cancer cells that persist in a patient's body during or after anti-cancer treatment when the patient is or appears to be in remission. These cancer cells cannot be detected by conventional diagnostic methods used to determine the treatment success of the anti-cancer treatment but can be detected by highly sensitive detection methods such as quantitative polymerase chain reaction (qPCR), next-generation flow cytometry (NGF) or next-generation sequencing (NGS). These cancer cells have the potential to cause relapse in the patient. In some embodiments, MRD refers to MRD of MM meaning to the small number of cancer cells associated with MM, e.g., malignant plasma cells, that persist in a patient's body during or after anti-cancer treatment when the patient is or appears to be in remission.

The terms "patient" and "subject" are used interchangeably herein and refer to a human of either gender (a male or a female). The subject may be of any age. In some embodiments, the subject is female. In some embodiments, the subject is male. In some embodiments, the subject has MM.

The term "target polynucleotide sequence" as used herein refers to a polynucleotide sequence that is specifically examined or manipulated in diagnostic test or molecular biology experiments. In some embodiments, the target polynucleotide sequence comprises or consist of a gene or a fragment thereof. In some embodiments, the target polynucleotide sequence comprises or consists of RNA. In some embodiments, the target polynucleotide sequence comprises or consists of DNA. In some embodiments, the target polynucleotide sequence comprises or consists of complementary DNA (cDNA). In some embodiments, the DNA sequence of the target polynucleotide sequence is amplified using PCR. In such embodiments, the target polynucleotide sequence is recognized by specific primers and DNA probes that bind to complementary sequences of the target polynucleotide sequence.

The term "target polynucleotide sequence corresponding to CXCR3" refers to a target polynucleotide sequence that comprises or consists of a coding sequence encoding CXCR3 or a fragment thereof or an isoform of CXCR3 or a fragment thereof.

The term "reference sequence polynucleotide" as used herein refers to a polynucleotide sequence that is specifically examined or manipulated in diagnostic test or molecular biology experiments and functions a reference to the target polynucleotide sequence. Usually, reference polynucleotide sequences encode proteins that are stably expressed in the biological sample. In some embodiments, the reference polynucleotide sequence comprises or consist of a gene or a fragment thereof. In some embodiments, the reference polynucleotide sequence comprises or consists of RNA. In some embodiments, the reference polynucleotide sequence comprises or consists of DNA. In some embodiments, the reference polynucleotide sequence comprises or consists of complementary DNA (cDNA). In some embodiments, the DNA sequence of the reference polynucleotide sequence is amplified using PCR. In such embodiments, the reference polynucleotide sequence is recognized by specific primers and DNA probes that bind to complementary sequences of the reference polynucleotide sequence. Examples of reference polynucleotide sequences are Glyceraldehyde 3-phosphate dehydrogenase (GADPH), Beta-Actin (ACTB), Beta-2-Microglobulin (B2M), Hypoxanthine Phosphoribosyltransferase 1 (HPRT1), Ribosomal Protein Lateral Stalk Subunit P0 (RPL0), TATA-Box Binding Protein (TBP), Glucuronidase Beta (GUSB), Tyrosine 3-Monooxygenase/Tryptophan 5-Monooxygenase Activation Protein, Zeta (YWAHZ), Phosphoglycerate Kinase 1 (PGK1), 18S rRNA, and Succinate Dehydrogenase Complex Flavoprotein Subunit A (SDHA).

The term "amplified product" as used herein refers to DNA amplified by PCR. In the context of the target polynucleotide sequence, the amplified product refers to the DNA sequence of the target polynucleotide sequence that is amplified by PCR. In some embodiments, the "amplified product" is also referred to as "amplicon".

The term "digital PCR" as used herein refers to a PCR in which the sample to be analyzed is divided into a large number (10,000 to 100,000) of small reaction volumes or reaction chambers (so-called "partitions" or "droplets"), in which a PCR process is carried out in parallel. In some embodiments, the digital PCR is a droplet digital PCR (ddPCR).

The term "DNA probe" as used herein refers to fluorescently labeled oligonucleotide probe used in PCR, particularly in digital PCR, for the specific detection and quantification of target polynucleotide sequences and reference polynucleotide sequences. The DNA probe consists of a short, single-stranded DNA sequence that is complementary to the target polynucleotide sequence or the reference polynucleotide sequence. Usually, the DNA probe is labeled with a fluorescent dye (reporter) at the 5' end and a quencher at the 3' end. Non limiting examples of DNA probes used herein are TaqMan probes, molecular beacons, and scorpion probes. In some preferred embodiments, the DNA probe is a TaqMan probe. During PCR amplification, the DNA probe binds specifically to the target polynucleotide sequence or the reference polynucleotide sequence. In the case of TaqMan probes, the DNA probes is degraded during the elongation phase of the PCR due to the 5'-3' exonuclease activity of the DNA polymerase. This results in the emission of a fluorescent signal that is suitable for detecting the target polynucleotide sequence or the reference polynucleotide sequence, e.g., in the in the partition of the digital PCR. In some embodiments, the DNA probe can be a target probe or a control probe, whereby it is preferred that the target probe binds to the sequence of the target polynucleotide sequence (e.g., a cDNA encoding CXCR3 or a fragment thereof or an isoform of CXCR3 or a fragment thereof) and the control probe binds to the reference polynucleotide sequence (e.g. a cDNA encoding GADPH or a fragment thereof). The term "quencher" as used herein refers to a molecule that absorbs the fluorescence energy of the fluorescent dye and thus prevents the emission of a fluorescent signal as long as the DNA probe is intact. The fluorescent dye and the quencher of the DNA probe can be selected from the fluorescent dyes and quenchers known in the art.

The terms "locked nucleic acid" or "LNA" are used interchangeably herein and refer to modified nucleic acid analogues which are stabilized by a chemical bridge between the 2'-O- and the 4'-C-atom of the ribose ring. This bridge fixes the ribose in a specific conformation, which improves the hybridization properties of the LNA, in particular leading to increased stability against chemical and enzymatic degradation, a high affinity for complementary DNA sequences and improved specificity in binding to the target sequence.

The term "enriching an immune cell fraction" as used herein refers to increasing the proportion of immune cells and/or specific immune cells in a biological sample. For example, the proportion of specific immune cells can be increased by selection and subsequent sorting of immune cells based on specific surface antigens of the immune cells, e.g., CD3, CD4, CD8, CD14, CD16, CD19, CD25, CD34, CD45RA, CD45RO, and CD56. For example, the selection and sorting of immune cells can be achieved by affinity purification techniques known to the skilled person.

The term "diagnosing" as us herein refers to the initial determination of the presence of a disease, disorder, or condition in a subject. In some embodiments, diagnosing refers to diagnosing of MM in a subject. Diagnosing of MM in a subject comprises the detection of MM in the subject.

The term "prognosing" as used herein refers to the prediction of the development and/or outcome of a disease, disorder, or condition in a subject. Prognosing as used herein also comprises adjusting the treatment plan of the subject based on the prognosis. In some embodiments, prognosing refers to prognosing of MM in a subject. Prognosing of MM in a subject comprises taking into account the following prognostic factors: classification of MM according to stages of the international staging system (ISS) and/or the revised international staging system (R-ISS), genetic abnormalities (e.g., del(17p), t(4;14), and t(14;16)), the response to treatment, in particular the subject's MRD status, patient related factors (e.g., age, overall health, gender, comorbidities), and biomarkers indicative of MRD.

The term "treatment monitoring" as used herein refers to the regular determination of the subject's response to the treatment of a disease, disorder, or condition, i.e., the treatment success. Treatment monitoring as used herein also comprises adjusting the treatment plan of the subject if needed. In some embodiments, treatment monitoring comprises "long term monitoring". The term "long term monitoring" as used herein refers to the determination of a subject's status of a disease, disorder, or condition once complete remission has been achieved. This means long term monitoring is performed to determine whether a disease, disorder, or condition is relapsing. In some embodiments, treatment monitoring refers to treatment monitoring of MM in a subject. In some embodiments, treatment monitoring of MM comprises the detection of the subject's MRD status.

The terms "limit of detection" or "LoD" are used interchangeably herein and refer to the lowest concentration of the target analyte, e.g., the amplicon of a PCR or a malignant cell, that can be reliably detected by the detection method. For example, a LoD of 10⁻⁶ means that the detection method can reliably detect 1 target analyte among 1,000,000 analytes.

### In vitro method for detecting multiple myeloma in a subject

The invention concerns an *in vitro* method for detecting multiple myeloma (MM) in a subject, wherein the method comprises:
a. obtaining a biological sample from the subject, and
b. determining the presence of the biomarker chemokine receptor CXCR3 (CXCR3) in the biological sample, wherein the presence of CXCR3 in the biological sample is indicative of MM in the subject.

CXCR3 is a class A G-protein-coupled receptor that is translocated at the cell membrane of T cells, including CD4⁺, CD8⁺ T cells and natural killer (NK) cells, but can also be found on neurons, astrocytes, endothelial cells and smooth muscle cells, as well as tumors. Playing crucial functions in angiogenesis, inflammation and cancer, Interferon-gamma inducible chemokines C-X-C motif chemokine ligand (CXCL) 4, CXCL9, CXCL10 and CXCL11 bind to CXCR3 to induce intracellular signaling by the regulation of MAPK/ERK and PI3 kinase/AKT signaling pathways. CXCR3 has three known isoforms, CXCR3-A, CXCR3-B, and CXCR3-alt, due to alternative splicing of the mRNA. CXCR3-A and CXCR3-B differ in their N-terminal extracellular domain. CXCR3-A is the splicing product of exon 1 and exon 3, whereas CXCR3-B has an additional exon 2 in between exon 1 and exon 3, making the variant 52 amino acids longer than CXCR3-A. CXCR3-alt is a truncated version of CXCR3-A with a shorter C-terminus and consists of five transmembrane domains instead of the seven transmembrane domains of CXCR3-A. CXCR3-alt is generated by posttranscriptional exon skipping.

The step of obtaining a biological sample from the subject is intended to refer to the provision of a sample that originates from the subject and is suitable for analysis. This includes receiving, collecting, isolating, or otherwise acquiring the biological sample, whether directly from the subject or indirectly from a previously collected source, as long as the sample is provided in a manner suitable for analytical processing. The act of obtaining the sample does not necessarily imply performance of a medical or surgical procedure by the person conducting the analysis.

The step of determining the presence of the biomarker CXCR3 in the biological sample refers to the *in vitro* detection, measurement, or qualitative or quantitative assessment of CXCR3 using analytical, biochemical, molecular, or computational methods. This process includes, but is not limited to, detecting the biomarker directly or indirectly through binding interactions, enzymatic reactions, signal detection, sequencing, or computational inference from obtained data. The term does not imply any *in vivo* diagnostic or therapeutic procedure and encompasses both manual and automated analytical approaches applied to an isolated sample.

In some embodiments, CXCR3 comprises or consists of at least one isoform of CXCR3. In some embodiments, CXCR3 comprises or consists of a isoform of CXCR3. In some embodiments, CXCR3 comprises or consists of CXCR3-A, CXCR3-B and/or CXCR3-alt. In some preferred embodiments, CXCR3 comprises or consists of CXCR3-A. In some embodiments, CXCR3 comprises or consists of CXCR3-B. In some embodiments, CXCR3 comprises or consists of CXCR3-alt. In the research underlying the invention, the applicant has surprisingly found that detecting an isoform of CXCR-3 such as CXCR3-A in a sample derived from MM subjects allows to detect MRD in the subject.

In some embodiments, the biological sample is derived from the blood of the subject. In some preferred embodiments, the biological sample is derived from the peripheral blood of the subject. In the context of the blood sample or peripheral blood sample, "derived from" means that the biological sample is obtained from the blood or peripheral blood and can be treated further before performing the *in vitro* detection methods described herein. For example, an immune cell fraction be enriched in the blood sample or peripheral blood sample. In some embodiments, the blood sample and/or peripheral blood sample can be a whole blood sample, i.e., a sample comprising red blood cells, white blood cells, platelets, and plasma.

Blood samples, particularly peripheral blood, offer significant advantages over other types of biological samples, such as bone marrow, for biomarker detection. Blood collection is minimally invasive, reducing patient discomfort and procedural risks compared to bone marrow aspiration, which requires an invasive procedure. Additionally, blood samples are easier to obtain, process, and store, enabling high-throughput analysis and facilitating longitudinal monitoring of biomarkers over time. Especially for assessing blood cell based diseases such as MM, peripheral blood also provides a systemic snapshot of circulating biomarkers, which may better reflect disease dynamics compared to localized bone marrow samples.

In some embodiments, the *in vitro* method for detecting MM in a subject comprises detecting MRD of MM in the subject. MRD is an important clinical parameter for evaluating treatment efficacy and predicting outcomes of MM subjects. The thresholds for categorizing subjects as MRD-positive, i.e., MRD is detected in the subject, and MRD-negative, i.e., MRD is not detected in the subject, depend on the method of detection used to detect the subject's MRD status and the clinical guidelines followed. Usually, the sensitivity of the method and the number of malignant cells detected in the biological sample are important factors for determining the subject's MRD status. In the following, non-limiting examples of MRD thresholds are provided:
- NGF: MRD-positive: presence of one malignant cell among 100,000 to 1,000,000 normal cells (sensitivity of 10⁻⁵ to 10⁻⁶); MRD-negative: no malignant cells are present among 100,000 to 1,000,000 normal cells.
- NGS: MRD-positive: presence of one malignant cell among 10,000,000 normal cells (sensitivity of 10⁻⁷); MRD-negative: no malignant cells are present among 10,000,000 normal cells.
- PCR: MRD-positive: presence of one malignant cell among 100,000 to 1,000,000 normal cells (sensitivity of 10⁻⁵ to 10⁻⁶); MRD-negative: no malignant cells are present among 100,000 to 1,000,000 normal cells.

In some embodiments, the *in vitro* method for detecting MRD of MM in a subject is a ddPCR method as described herein. Compared to the NGF gold standard method for the detection of MRD of MM, the ddPCR method described herein does not directly detect malignant cells in the bone marrow, but detects an amplified immune response triggered by myeloma cells in the bone marrow, measurable in the peripheral blood. This amplified immune response is reflected by shifted expression of CXCR3 in cells e.g. comprised in the peripheral blood, particularly by an increased expression of CXCR3-A on the surface of T cells. Without wishing to be bound by theory, it is contemplated herein that since one myeloma cell can modulate multiple T cells, the ddPCR method described herein can leverage an amplified signal rather than detecting individual malignant cells. Therefore, the determination of thresholds for MRD positivity and negativity is different from the NGF gold standard method. In the research underlying the invention, it was found that the MRD thresholds for the ddPCR method can be determined based on the detection of CXCR3-A in the peripheral blood of healthy donors (cf. example 1). In healthy donors, the ratio between CXCR3-A and the reference polynucleotide sequence GADPH was < 0.01 ± 10%. The range 0.01 ± 10% is intended to cover values between 0.009 and 0.011.

In some embodiments, the threshold for classifying a subject as MRD-positive in the ddPCR method as described herein is a ratio of CXCR3A to GADPH >0.01 ± 10%.

In some embodiments, the threshold for classifying a subject as MRD-negative in the ddPCR method as described herein is a ratio of CXCR3A to GADPH <0.01 ± 10%.

The skilled person knows the concept of MRD, particularly with respect to MRD of MM, and can readily categorize a subject as MRD-positive or MRD-negative when provided with a suitable detection method and a biological sample.

The method for determining the presence or detecting the presence of CXCR3 in the biological sample is not limited to specific detection methods. In some embodiments, CXCR3 is detected at the protein and/or nucleic acid level. In some embodiments, CXCR3 is detected at the protein level. In such embodiments, the CXCR3 protein or an isoform thereof is directly detected, e.g., on the surface of T cells.

Methods for detecting CXCR3 at the protein level include but are not limited to enzyme-linked immunosorbent assay (ELISA), single molecule arrays (SiMoA), immunohistochemistry (IHC), mass spectrometry (MS), flow cytometry, immunoprecipitation, protein microarray, proximity extension assay, and/or western blot.

In some preferred embodiments, CXCR3 is detected at the nucleic acid level. In some preferred embodiments, CXCR3-A is detected at the nucleic acid level. For example, RNA such as mRNA encoding CXCR3 or an isoform thereof can be extracted from a biological sample and converted to cDNA by reverse transcription. The specific polynucleotide sequence of the cDNA encoding CXCR3 can then be detected by any suitable method.

Methods for detecting CXCR3 at the nucleic acid level include but are not limited to polymerase chain reaction (PCR), loop-mediated isothermal amplification (LAMP), next-generation sequencing (NGS), *in-situ* hybridization, nucleic acid microarray, CRISPR-Cas-based RNA detection, and/or northern blot.

In some preferred embodiments, CXCR3 is detected at the nucleic acid level by PCR.

In some embodiments, the determining in step b. comprises amplifying, using a PCR assay, a target polynucleotide sequence corresponding to CXCR3 in the biological sample, thereby generating an amplified product.

In some embodiments, the PCR assay is selected from a digital PCR (dPCR) and a real-time PCR (qPCR). In some embodiments the PCR assay is a qPCR. In some preferred embodiments the PCR assay is a dPCR. In some more preferred embodiments, the PCR assay is a droplet digital PCR (ddPCR). The advantages of ddPCR methods, in particular over qPCR methods, are absolute quantification by measuring the exact copy numbers of the amplicons, high sensitivity by partitioning the reaction into thousands of nanoliter-sized droplets or partitions thereby enabling the detection of subtle expression differences, and improved reproducibility by minimizing amplification bias as each droplet or partition serves as an independent reaction.

In some embodiments, the PCR or the PCR assay is performed with primers and one or more DNA probes. In the context of primers and DNA probes, the plural refers to different types of primers and/or DNA probes and not the specific number of primers and/or DNA probes present in the reaction.

In some embodiments, the primers have, independently of one another, a length of 15 to 30 nucleotides. In some embodiments, the primers have, independently of one another, a length of 18 to 27 nucleotides. In some embodiments, the primers have, independently of one another, a length of 15, 16, 17, 18, 19, 20, 21, 22, 23, 24, 25, 26, 27, 28, 29, or 30 nucleotides. In some preferred embodiments, the primers have a length of 20 nucleotides.

In some embodiments, the DNA probes have, independently of one another, a length of 10 to 35 nucleotides. In some embodiments, the DNA probes have, independently of one another, a length of 13 to 30 nucleotides. In some embodiments, the DNA probes have, independently of one another, a length of 10, 11, 12, 13, 14, 15, 16, 17, 18, 19, 20, 21, 22, 23, 24, 25, 26, 27, 28, 29, 30, 31, 32, 33, 34, or 35 nucleotides. In some preferred embodiments, the DNA probes have a length of 20 nucleotides.

In some embodiments, the primers and/or the one or more DNA probes comprise or consist of locked nucleic acids. In some embodiments, the one or more DNA probes comprise or consist of locked nucleic acids. In some preferred embodiments, the primers comprise or consist of locked nucleic acids. In some embodiments, an individual primer comprises 1, 2, 3, 4, 5, 6, 7, 8, 9, or 10 locked nucleic acids. In some preferred embodiments, an individual primer comprises 4-6, e.g., 4, locked nucleic acids.

In some embodiments, the DNA probe is labelled with a fluorescent dye and a quencher. In some embodiments, the DNA probe is labelled with a fluorescent dye at the 5' terminus and with a quencher at the 3' terminus. Non-limiting examples of fluorescent dyes suitable for the DNA probe described herein are 6-carboxyfluorescein (FAM), Hexachloro-fluorescein (HEX), Carboxy-X-rhodamine (ROX), 2'-chloro-7'-phenyl-1,4-dichloro-6-carboxyfluorescein (VIC), Cyanine 5 (Cy5), Tetrachloro-fluorescein (TET), 2',7'-Dimethoxy-4',5'-dichloro-6-carboxyfluorescein (JOE), and Texas Red. Non limiting examples of quenchers suitable for the DNA probe as described herein are Tetramethylrhodamine (TAMRA), Black Hole Quencher-1 (BHQ1), Black Hole Quencher-2 (BHQ2), 4-(4'-Dimethylaminophenylazo)benzoic acid (DABCYL), QSY 7, QSY 21, Iowa Black FQ, Iowa Black RQ, Eclipse Dark Quencher, Deep Dark Quencher-I (DDQ-I), and Deep Dark Quencher-II (DDQ-II).

In some embodiments, the DNA probe is labelled with FAM at the 5' terminus and with BHQ1 at the 3' terminus. In some preferred embodiments, the target probe is labelled with FAM at the 5' terminus and with BHQ1 at the 3' terminus.

In some preferred embodiments, at least one of the DNA probes is capable of binding to the target polynucleotide sequence. In some embodiments, the PCR is performed with a target probe.

In some embodiments, the target probe is capable of binding to the target polynucleotide sequence corresponding to CXCR3. For example, the target probe is capable of binding to a cDNA encoding CXCR3 or an isoform thereof, preferably to isoform A of CXCR3. Thereby the presence of the target polynucleotide sequence corresponding to CXCR3 in the biological sample can be detected once the target probe emits the fluorescent signal during PCR amplification upon degradation of the target probe.

In some preferred embodiments, the target probe comprises or consists of the sequence set forth in SEQ ID NO: 6 or a sequence having at least 60%, at least 70%, at least 80%, or at least 90% identity to said SEQ ID NO.

In some embodiments, the PCR is performed with a first primer pair which is capable of amplifying the target polynucleotide sequence and with a first DNA probe which is capable of binding to the target polynucleotide sequence.

In some preferred embodiments, the PCR is performed with a first primer pair which is capable of amplifying the target polynucleotide sequence corresponding to CXCR3 and with a first DNA probe which is capable of binding to the target polynucleotide sequence corresponding to CXCR3. For example, the PCR is performed with a forward and a reverse primer which are capable of amplifying the target polynucleotide sequence corresponding to CXCR3 and with a target probe which is capable of binding to the target polynucleotide sequence corresponding to CXCR3.

In some embodiments, the target polynucleotide sequence corresponding to CXCR3 comprises or consists of a coding sequence encoding CXCR3 or an isoform thereof. In some preferred embodiments, the target polynucleotide sequence corresponding to CXCR3 comprises or consists of a coding sequence encoding CXCR3-A.

In some embodiments, the target polynucleotide sequence corresponding to CXCR3-A comprises or consists of the sequence set forth in SEQ ID NO: 1 or a sequence having at least 60%, at least 70%, at least 80%, or at least 90% identity to said SEQ ID NO.

The primer pair can be designed to specifically amplify the target polynucleotide sequence corresponding to CXCR3-A. For example, this can be achieved by targeting both exon 1 and exon 3 of CXCR3 with one of the primers, e.g., with the forward primer. To target both exon 1 and exon 3 of CXCR3 at the same time, the primer can be designed to bind both to the DNA sequence of exon 1 and the DNA sequence of exon 3 of CXCR3. Preferably, the primer is designed to bind to a continuous stretch of polynucleotides in the target polynucleotide sequence corresponding to CXCR3 which comprises both a part of the DNA sequence of exon 1 and a part of the DNA sequence of exon 3. In other words, the primer is designed to traverse the junction between exon 1 and exon 3 of CXCR3. Thereby, the primer pair is only capable of amplifying isoform A of CXCR3, while isoforms B and alt are not amplified. It is generally preferred that the forward primer targets exon 1 and exon 3 of CXCR3.

In some preferred embodiments, the forward primer capable of amplifying the target polynucleotide sequence corresponding to CXCR3-A comprises or consists of the sequence set forth in SEQ ID NO: 4 or a sequence having at least 60%, at least 70%, at least 80%, or at least 90% identity to said SEQ ID NO.

In some preferred embodiments, the reverse primer capable of amplifying the target polynucleotide sequence corresponding to CXCR3-A comprises or consists of the sequence set forth in SEQ ID NO: 5 or a sequence having at least 60%, at least 70%, at least 80%, or at least 90% identity to said SEQ ID NO.

In some embodiments, the PCR is performed with a second primer pair capable of amplifying a reference polynucleotide sequence and with a second DNA probe capable of binding to the reference polynucleotide sequence.

In some preferred embodiments, the PCR is performed with a second primer pair capable of amplifying a GAPDH reference polynucleotide sequence and with a second DNA probe, e.g., a control probe, capable of binding to the GAPDH reference polynucleotide sequence. Primer pairs and control probes targeting GADPH are well known in the art and are commercially available. For example, a GADPH kit suitable for ddPCR can be obtained from Biorad Laboratories GmbH, Feldkirchen, Germany (ddPCR^{™} Gene Expression Assay: Gapdh, Mouse, Mus musculus).

In some embodiments, the first and the second primer pair are present in the same PCR reaction. In some embodiments, the first and the second DNA probe are present in the same PCR reaction. In these embodiments, the *in vitro* method has the advantage of being easy to set up and carry out and, in particular, can be provided in the form of a kit with a few components.

In some preferred embodiments, the target polynucleotide sequence corresponding to CXCR3-A is amplified by a ddPCR assay comprising:
- a first primer pair consisting of a forward primer comprising or consisting of the sequence set forth in SEQ ID NO: 4 or a sequence having at least 60%, at least 70%, at least 80%, or at least 90% identity to said SEQ ID NO, and a reverse primer comprising or consisting of the sequence set forth in SEQ ID NO: 5 or a sequence having at least 60%, at least 70%, at least 80%, or at least 90% identity to said SEQ ID NO;
- a target probe comprising or consisting of the sequence set forth in SEQ ID NO: 6 or a sequence having at least 60%, at least 70%, at least 80%, or at least 90% identity to said SEQ ID NO;
- a second primer pair consisting of a forward and reverse primer capable of amplifying the GADPH reference polynucleotide sequence; and
- a control probe capable of amplifying the GADPH reference polynucleotide sequence.

In some embodiments, *in vitro* method for detecting MM in a subject has a limit of detection (LoD) of 10⁻⁵ to 10⁻⁶. In some embodiments, *in vitro* method for detecting MM in a subject has an LoD of 10⁻⁶. In some embodiments, *in vitro* method for detecting MM in a subject has an LoD of 10⁻⁵.

In some embodiments, the *in vitro* method for detecting MM, particularly MRD of MM, in a subject is a ddPCR method. In some embodiments, the ddPCR method for detecting MM, particularly MRD of MM, in a subject has an LoD of 10⁻⁵ to 10⁻⁶. In some embodiments, the ddPCR method for detecting MM, particularly MRD of MM, in a subject has an LoD of 10⁻⁶. In some embodiments, the ddPCR method for detecting MM, particularly MRD of MM, in a subject has an LoD of 10⁻⁵.

Compared to the NGF gold standard method for the detection of MRD of MM, the ddPCR method described herein does not directly detect malignant cells in the bone marrow, but detects an amplified immune response triggered by myeloma cells in the bone marrow, measurable in the peripheral blood. This amplified immune response is reflected by shifted expression of CXCR3 in cells comprised in the peripheral blood, particularly by an increased expression of CXCR3-A on the surface of T cells. Therefore, the determination of the LoD for the ddPCR method described herein is different from the NGF gold standard method. In the research underlying the invention, it was found that the results of the CXCR3-A ddPCR assay correlated with the results of an established NGF method (cf. example 1). Without wishing to be bound by theory, it is contemplated herein that the CXCR3-A ddPCR method described herein has at least the same LoD as the NGF methods.

In some embodiments, between steps a. and b. of the *in vitro* detection method described herein, step a.1 is performed, which comprises enriching an immune cell fraction in the biological sample. In some preferred embodiments, T cells are enriched in the biological sample. In some more preferred embodiments, CD3+ T cells are enriched in the biological sample.

Enriching an immune cell fraction in the biological sample, e.g., the blood or peripheral blood sample as described herein, has the advantage of reducing the background of the detection method by preventing non-specific detection of CXCR3 or an isoform thereof. In particular, for ddPCR-based detection of CXCR3-A, the applicant surprisingly found that enriching T cells, particularly CD3+ T cells, in the biological sample increases the sensitivity, specificity, precision, and accuracy of the ddPCR assay. Without wishing to be bound by theory, it is contemplated herein that this increase in sensitivity, specificity, precision, and accuracy is due to the reduced number of other cells expressing CXCR3 in the biological sample. Methods for enriching a immune cell fraction in a biological sample are well known in the art and include density gradient centrifugation, magnetic-activated cell sorting (MACS), fluorescence-activated cell sorting (FACS), and immunomagnetic separation.

The invention also provides the use of the *in vitro* method as described herein for diagnosing, prognosing, and/or treatment monitoring of multiple myeloma in a subject.

In some embodiments, the *in vitro* method as described herein is used for diagnosing MM in a subject. For example, the *in vitro* method as described can be used to determine whether or not a subject is afflicted with MM.

In some embodiments, the *in vitro* method as described herein is used for prognosing MM in a subject. For example, a subject afflicted with MM is treated by, e.g., chemotherapy, immunotherapy, CAR-T cell therapy, radiation therapy, corticosteroids, bisphosphonates, and/or stem cell transplants. In the following, the subject's response to the treatment is monitored, including the subject's MRD status. Taking the subject's response to the treatment and additional prognostic markers such as classification according the international staging system (ISS) and/or the revised international staging system (R-ISS), genetic abnormalities (e.g., del(17p), t(4;14), and t(14;16)), patient related factors (e.g., age, overall health, gender, comorbidities), and biomarkers indicative of MRD into account, the development of MM in the subject and/or the overall subject outcome is predicted. The *in vitro* method as described herein is particularly useful for prognosing of MM in a subject because it can detect MM in a subject, particularly the subject's MRD status, with high with high sensitivity, specificity, precision, and accuracy. Furthermore, the *in vitro* method as described herein allows the subject's MRD status to be detected in the subject's peripheral blood, allowing for frequent testing of the subject's MRD status with minimal pain and discomfort to the subject. This is particularly advantageous compared to state-of-the-art methods such as NGF and NGS, which detect the subject's MRD status based on bone marrow aspirates.

In some embodiments, the *in vitro* method as described herein is used for treatment monitoring of MM in a subject. In particular, the convenient detection of the subject's MRD status in the peripheral blood is useful for the treatment monitoring and the long term monitoring as described herein because it allows frequent testing of the subject's MRD status with minimal pain and discomfort to the subject.

The embodiments for an *"in vitro* method for detecting MM in a subject" as described herein can be used in the embodiments relating to the "use of the *in vitro* method for diagnosing, prognosing, and/or treatment monitoring of MM in a subject". Features described herein in more detail in connection with the *"in vitro* method for detecting MM in a subject" embodiments equally apply to the corresponding "use of the *in vitro* method for diagnosing, prognosing, and/or treatment monitoring of MM in a subject" embodiments.

### CXCR3 as a biomarker for diagnosing, prognosing, and/or treatment monitoring of multiple myeloma

The invention further concerns the use of chemokine receptor CXCR3 (CXCR3) as a biomarker for diagnosing, prognosing, and/or treatment monitoring of multiple myeloma (MM) in a subject.

In some embodiments, CXCR3 is used as a biomarker for diagnosing of MM in a subject. In some embodiments, CXCR3 is used as a biomarker for prognosing of MM in a subject. In some embodiments, CXCR3 is used as a biomarker for treatment monitoring of MM in a subject. In these embodiments, the detection of the biomarker CXCR3 is indicative of MM in the subject.

In some embodiments, CXCR3 is used as a biomarker to detect MRD of MM in the subject. MRD is an important clinical parameter for evaluating treatment efficacy and predicting outcomes of MM subjects.

In some embodiments, CXCR3 comprises or consists of at least one isoform of CXCR3. In some embodiments, CXCR3 comprises or consists of one isoform of CXCR3. In some embodiments, CXCR3 comprises or consists of CXCR3-A, CXCR3-B and/or CXCR3-alt. In some preferred embodiments, CXCR3 comprises or consists of CXCR3-A. In some embodiments, CXCR3 comprises or consists of CXCR3-B. In some embodiments, CXCR3 comprises or consists of CXCR3-alt. The applicant has demonstrated that CXCR3-A can be used to detect MRD in the peripheral blood of patients afflicted with MM. In particular, when expressed on the surface of T cells, in particular CD3+ T cells, CXCR3-A can be used as a biomarker to detect MRD of MM in the peripheral blood of subjects.

In some preferred embodiments, isoform A of the biomarker CXCR3 is encoded by the sequence comprising or consisting of the sequence set forth in SEQ ID NO: 1 or a sequence having at least 60%, at least 70%, at least 80%, or at least 90% identity to said SEQ ID NO.

In some embodiments, the biomarker CXCR3 is detected in a biological sample derived from the blood of the subject. In some preferred embodiments, the biomarker CXCR3 is detected in the peripheral blood of the subject.

The detection of the biomarker CXCR3 is not limited to a specific detection method.

In some embodiments, CXCR3 is detected at the protein and/or nucleic acid level. In some embodiments, CXCR3 is detected at the protein level. In such embodiments, the CXCR3 protein or an isoform thereof is directly detected, e.g., on the surface of T cells.

Methods for detecting CXCR3 at the protein level include but are not limited to enzyme-linked immunosorbent assay (ELISA), single molecule arrays (SiMoA), immunohistochemistry (IHC), mass spectrometry (MS), flow cytometry, immunoprecipitation, protein microarray, proximity extension assay, and/or western blot.

In some preferred embodiments, CXCR3 is detected at the nucleic acid level. In some preferred embodiments, CXCR3-A is detected at the nucleic acid level. For example, RNA such as mRNA encoding CXCR3 or an isoform thereof can be extracted from a biological sample and converted to cDNA by reverse transcription. The specific polynucleotide sequence of the cDNA encoding CXCR3 can then be detected by any suitable method.

Methods for detecting CXCR3 at the nucleic acid level include but are not limited to polymerase chain reaction (PCR), loop-mediated isothermal amplification (LAMP), next-generation sequencing (NGS), *in-situ* hybridization, nucleic acid microarray, CRISPR-Cas-based RNA detection, and/or northern blot.

In some preferred embodiments, the biomarker CXCR3 is detected by PCR. In some embodiments, the PCR is selected from dPCR and qPCR. In some embodiments the biomarker CXCR3 is detected by qPCR. In some preferred embodiments, the biomarker CXCR3 is detected by dPCR. In some more preferred embodiments, the biomarker CXCR3 is detected by ddPCR.

The embodiments for an *"in vitro* method for detecting MM in a subject" and the "use of the *in vitro* method for diagnosing, prognosing, and/or treatment monitoring of MM in a subject" as described herein can be used in the embodiments relating to the "use of CXCR3 as a biomarker for diagnosing, prognosing, and/or treatment monitoring of MM in a subject".

Features described herein in more detail in connection with the *"in vitro* method for detecting MM in a subject" and the "use of the *in vitro* method for diagnosing, prognosing, and/or treatment monitoring of MM in a subject" embodiments equally apply to the corresponding "use of CXCR3 as a biomarker for diagnosing, prognosing, and/or treatment monitoring of MM in a subject" embodiments.

### Kit for the detection of multiple myeloma

The invention further provides a kit for the detection of multiple myeloma in a subject, wherein the kit comprises:
- a first primer pair which is capable of amplifying a target polynucleotide sequence corresponding to the biomarker chemokine receptor CXCR3, and
- a first DNA probe which is capable of binding to the target polynucleotide sequence.

In some embodiments, the kit further comprises
- a second primer pair which is capable of amplifying a reference polynucleotide sequence, and
- a second DNA probe which is capable of binding to the reference polynucleotide sequence.

The embodiments for an *"in vitro* method for detecting MM in a subject", the "use of the *in vitro* method for diagnosing, prognosing, and/or treatment monitoring of MM in a subject", and the "use of CXCR3 as a biomarker for diagnosing, prognosing, and/or treatment monitoring of MM in a subject" as described herein can be used in the embodiments relating to the "kit for the detection of MM in a subject".

Features described herein in more detail in connection with the *"in vitro* method for detecting MM in a subject", the "use of the *in vitro* method for diagnosing, prognosing, and/or treatment monitoring of MM in a subject", and the "use of CXCR3 as a biomarker for diagnosing, prognosing, and/or treatment monitoring of MM in a subject" embodiments equally apply to the corresponding "kit for the detection of MM in a subject" embodiments.

### SEQUENCE LISITING

This application contains a Sequence Listing which has been submitted electronically and is hereby incorporated by reference in its entirety. Said Sequence Listing file is named 241262EP_sequence_listing.xml and 12.204 Bytes in size.
SEQ ID NO: 1 is an exemplary polynucleotide sequence of CXCR3-A. Nucleotides 1-74 represent exon 1 of CXCR3. Nucleotides 75-1615 represent exon 3 of CXCR3.
SEQ ID NO: 2 is an exemplary polynucleotide sequence of CXCR3-B. Nucleotides 1-73 represent exon 1 of CXCR3. Nucleotides 74-317 represent exon 2 of CXCR3. Nucleotides 318-1861 represent exon 3 of CXCR3.
SEQ ID NO: 3 is an exemplary polynucleotide sequence of the amplified product obtained by the *in vitro* CXCR3-A ddPCR assay described herein.
SEQ ID NO:4 is an exemplary polynucleotide sequence of a forward primer. The forward primer comprises locked nucleic acids at polynucleotide positions 8, 9, 10, and 14 in 5' to 3' orientation.
SEQ ID NO: 5 is an exemplary polynucleotide sequence of a reverse primer.
SEQ ID NO: 6 is an exemplary polynucleotide sequence of a target probe. The DNA probe is labelled with FAM at the 5' terminus and with BHQ1 at the 3' terminus.

### EXAMPLES

### Materials and methods

The following materials and methods were used to perform the experiments described below.

### Blood samples

Peripheral blood (PB) samples from MRD-positive MM patients were included in accordance with the Declaration of Helsinki and approved by the Ethics Committee of the Medical Faculty at Heidelberg University. Peripheral blood samples from the Central laboratory, Heidelberg, Germany, from MRD-positive MM patients were used as positive control. Buffy coats from healthy donors from the Institute for Immunology/IKTZ, Heidelberg University, Germany were used as negative control.

### Cell isolation

Frozen PB patient samples from the GMMG-HD7 clinical trial were thawed in RPMI medium (Sigma-Aldrich, #R8758-500ML, Taufkirchen, Germany), supplemented with 20 % fetal bovine serum (FBS) (Gibco, #A5256701, Darmstadt, Germany). Cells were counted with LUNA and sorted for negative CD3⁺ selection using Pan T cell isolation kit (Miltenyi, #130-096-535, Gladbach, Germany) according to manufacturer's protocol. Total cells were centrifuged at 350xg, 6 min, supernatant was discarded, and cells were resuspended in 40 µl MACS buffer (Phospate buffered saline PBS (Sigma-Aldrich, #D8537-500ML, Taufkirchen, Germany) + 1 mM EDTA). 10 µl Pan T cell biotin-antibody cocktail per 1 x 10⁷ total cells were added, mixed and incubated for 5 min at 4 °C. After incubation, 30 µl MACS buffer and 20 µl Pan T cell microbeads per 1 x 10⁷ cells were added, mixed and incubated for 10 min at 4 °C. During 10 min incubation, LD magnetic column (Miltenyi, #130-042-901, Mönchengladbach, Germany) was prewashed with 2 ml MACS buffer. After 10 min incubation, the cell suspension was applied onto the column and the flow-through was collected containing unlabeled cells in 15 ml tubes (Sarstedt, #62.547.254, Nümbrecht, Germany), representing the enriched CD3⁺ T cells. Columns were washed with 2 ml MACS buffer twice. Cells that passed through during the washing steps were further collected and combined with all effluents from previous steps. Unlabeled cells were centrifuged at 350xg, 6 min and resuspended in 1 ml PBS each. CD3⁺ T cells were counted using LUNA and dry pellets from each sample were collected after centrifugation at 2500xg for 5 min. PBS was discarded, and dry pellets were stored at - 80 °C immediately.

For samples from the Central laboratory, Heidelberg, Germany, CD3⁺ T cells were directly isolated from the blood by positive CD3⁺ T cell isolation using EasySep HLA Chimerism Whole Blood CD3 Positive Selection Kit (Stemcell Technologies, #17871, Cologne, Germany), according to manufacturer's protocol. 10X EasySep RBC Lysis buffer (provided in the kit) was diluted 1:10 in double distilled water in order to obtain 1X lysis buffer. 2.5 ml blood sample was transferred into a 15 ml polystyrene round-bottom tube (Falcon, #352052, Berlin, Germany) and 2.5 ml 1X lysis buffer was added to the sample. 250 µl selection cocktail (25 µl/ml end concentration, provided in the kit) of diluted sample were added, mixed and incubated for 3 min at room temperature. During incubation, RapidSpheres (25 µl/ml end concentration, provided in the kit) were vortexed for 30 s and 250 µl were added immediately, mixed and incubated for 3 min at room temperature. 7 ml washing buffer (PBS containing 2 % FBS with 1 mM EDTA) were topped up, mixed by gently pipetting up and down two times, placed into the magnet and further incubated for 10 min at room temperature. Supernatant was pipetted off without touching the tube wall. Cells were washed two times with 7 ml washing buffer, following the described steps as above. After washing, tubes were removed from the magnet and cells resuspended in 1 ml PBS. Cell suspension was transferred into a 1.5 ml sterile tube and counted using LUNA. Dry pellets were collected from each patient sample by centrifugation at 2500xg, 5 min, and subsequent discarding PBS. All samples were stored in -80 °C.

### RNA isolation

Dry pellets were resuspended in 1 ml Trizol (Life technologies, #15596026, Darmstadt, Germany) and incubated for 5 min at room temperature. 200 µl chloroform (Sigma-Aldrich, #366919-1L, Taufkirchen, Germany) was added, vortexed for 15 s and incubated for 2 min at room temperature. Samples were centrifuged at 13000xg for 5 min at 4°C. Afterwards, transparent phase containing RNA were collected in a new tube. 500 µl isopropanol were added and samples were gently mixed. After 15 min incubation at room temperature, samples were centrifuged at 13000xg for 10 min at 4 °C and supernatant were carefully removed. RNA extraction was further continued using Rneasy Plus Mini Kit (Qiagen, #74134, Hilden, Germany), according to manufacturer's protocol. Hereby, protein digestion was achieved by adding 15 µl proteinase K solution (provided in the kit) to the precipitation and incubated for 15 min at room temperature. 400 µl RW1 buffer (provided in the kit) were added and mixed briefly by inverting the tubes. 200 µl RW1 buffer were added to Rneasy spin colum (provided in the kit) for priming before the entire solution was transferred to the spin column with total volume of 700 µl and centrifuged for 30 s at 8000xg. Flow-through was discarded and the previous step was repeated as soon as the whole sample was loaded on the column. Washing was performed, applying 400 µl RW1 buffer to the column and subsequent centrifugation for 30 s at 8000xg. 10 µl reconstituted PureLink DNase was combined with 40 µl 2X DNase I reaction buffer (ThermoFisher, #12185010, Darmstadt, Germany) and applied to the column, following 30 min incubation at room temperature. Column was washed with 500 µl RW1 buffer, centrifuged for 15 s at 8000xg and further washed with RPE buffer (provided in the kit) with 2 min centrifugation at 8000xg. Columns were dried using centrifugation at 13000xg for 5 min. Rneasy spin column (provided in the kit) were placed in a sterile 1.5 ml tube and 30 µl RNAse-free water was directly pipetted on the column. After 1 min centrifugation at 8000xg, RNA was eluted and measured using Quantus Fluorometer with RNA dye (Promega, #E3310, Walldorf, Germany). RNA was stored in -80°C until further use.

### Measuring of RNA concentration

RNA concentration was measured using QuantiFluor RNA System kit (Promega, #E3310, Walldorf, Germany) and detected by Quantus Fluorometer (Promega, Walldorf, Germany). For calibration, 20X TE buffer (provided in the kit) was diluted 1:20 with nuclease-free water in order to obtain 1X TE buffer. RNA dye was diluted 1:400 in 1X TE buffer and blank sample was measured using 200 µl diluted dye in a 0.5 ml Quantus tube (provided in the kit). RNA standard (provided in the kit) was diluted 1:100 in 1X TE buffer and 10 µl diluted sample with 200 µl diluted dye were measured after blank calibration. Standard calibration was saved, and patients' samples were prepared. Hereby, 1 µl RNA patient sample was mixed with 200 µl diluted dye in a 0.5 ml Quantus tube (Promega, #E4942, Walldorf, Germany). After 5 min incubation in the dark at room temperature, RNA samples were measured with Quantus Fluorometer.

### cDNA synthesis

cDNA was synthesized based on extracted RNA of patients' samples. 1 µg RNA was prepared in total volume of 10 µl nuclease free water and incubated at 4 °C before immediate use. In case the extracted RNA amount did not exceed 1 µg, 10 µl undiluted RNA was used. Master mix was pipetted in an extra tube, containing the following components from the High Capacity cDNA Reverse Transcription Kit (Applied Biosystems, #4368814, Darmstadt, Germany). For one reaction, the following volumes for each component were used: 4.2 µl nuclease free water, 2 µl 10X RT buffer, 0.8 µl 25X dNTPs, 2 µl 10X random primer, 50 U multiScribe reverse transcriptase. 10 µl master mix was added to diluted RNA sample in PCR tubes, mixed and synthesized in a thermocycler (Analytik Jena, Jena, Germany), using the following program: 25 °C 10 min; 37 °C 2X 60 min; 85 °C 5 min. cDNA was kept hold at 16 °C before further use or was stored at -20 °C for long time storage.

### Digital PCR

The primers and DNA probes for the CXCR3-A ddPCR assay were synthesized by Integrated DNA Technologies, Munich, Germany. For reference, a ddPCR assay targeting GAPDH with a 6-carboxy-2 ,4,4,5 ,7,7 -hexachlorofluorescein succinimidyl ester (HEX) DNA probe was bought from Biorad Laboratories GmbH, Feldkirchen, Germany. Master Mix for ddPCR was prepared using the following components: 5 µl 4X ddPCR Multiplex Supermix (Biorad, #12005910, Feldkirchen, Germany), 1µl 20X CXCR-3 assay mix, 1 µl 20X GAPDH primers and HEX DNA probe, 8 µl RNAse/DNAse free water and cDNA synthesis mix, calculated for 5 ng initial RNA. From each sample, duplicates with 20 µl sample volume were carefully pipetted into individual wells of a DG8^{™} cartridge (Biorad Laboratories, #1864008, Feldkrichen, Germany), and measured with a QX200 Droplet Generator (Biorad Laboratories, Feldkrichen, Germany). 70 µl Droplet Generation Oils (Biorad Laboratories, #1863005, Feldkrichen, Germany) were added to the cartridge and sealed with a gasket (Biorad Laboratories, #1863009, Feldkrichen, Germany) afterwards. The QX200 Droplet Generator was closed until droplets were formed. Cartridge and gasket were carefully removed and 40 µl of the droplet solution (not the upper cloudy phase) was transferred into a 96 well PCR plate (Biorad, #12001925, Feldkirchen, Germany). The PCR plate was placed into the PX1 PCR Plate Sealer (Biorad, Feldkirchen, Germany) to cover the plate, and the ddPCR was performed in a thermo cycler (C1000 Touch Thermal Cycler, Biorad Laboratories, Feldkirchen, Germany), using the following program: Enzyme activation at 95 °C for 10 min, 40 cycles of denaturation at 94 °C for 30 seconds and annealing/extension at 57 °C for 1 min; after 40 cycles, enzyme deactivation was performed at 98 °C for 10 min. Samples were kept on hold afterwards at 4 °C.

ddPCR results were analyzed using QuantaSoft (Biorad, Feldkirchen, Germany) to determine GAPDH and CXCR3 transcript numbers and calculate CXCR3 to GAPDH ratios. The baseline of CXCR3-A high to low was calculated based on negative controls.

NGF was performed according to the highly standardized multicolor flow cytometry approach developed and described by the Spanish Myeloma Collaborative Group using a commercially available EuroFlow 8-color 2-tube MM MRD Kit (Cytognos, BD) according to manufacturing protocol.

### Example 1: CXCR3-A ddPCR assay for detecting MRD of MM in the peripheral blood of a subject

To investigate whether the ddPCR assay of the invention was suitable for determining the MRD status of a MM subject, peripheral blood (PB) samples from MRD-positive and MRD-negative MM subjects and healthy donors (negative control) were analyzed using the CXCR3-A ddPCR assay. The MRD status of the subjects was determined using state of the art Next-Generation-Flow (NGF) analysis of bone marrow aspirates. cDNA of CXCR3-A negative expressed human acute myeloid leukemia cell line HL-60, incubated with different concentrations of synthesized CXCR3-A assay IDT sequence was used as a positive control.

For the CXCR3-A ddPCR assay, CD3+ T cells were enriched in the PB samples, as described in the section "cell isolation" above, before performing the ddPCR reaction. A forward primer (comprising locked nucleic acids; SEQ ID NO: 4) and a reverse primer (SEQ ID NO: 5) were designed to amplify the cDNA sequence of CXCR3-A (SEQ ID NO: 1). The amplicon of the CXCR3-A ddPCR assay is shown in SEQ ID NO: 3. A target probe (SEQ ID NO: 6) labelled with FAM at the 5' terminus and with BHQ1 at the 3' terminus was designed to bind to the cDNA sequence of CXCR3-A. The ddPCR was performed as described in section "digital PCR" above.

The following parameters were applied for the CXCR3-A ddPCR assay
- Droplet threshold: at least 10,000 droplets per well were used.
- RNA concentration for cDNA synthesis: at most 1 µg RNA was used per cDNA synthesis reaction.
- GADPH reference polynucleotide sequence: a threshold of at least 2,000 GADPH transcripts per well was used.

The threshold for CXCR3-A detection was determined based on peripheral blood samples from healthy donors (n=9). In healthy donors, the ratio between CXCR3-A and GADPH was < 0.01 ± 10%. Therefore, the threshold for classifying a subject as MRD-positive was set to a ratio of CXCR3-A to GADPH of >0.01 ± 10%.

The results depicted in table 1 below show that the CXCR3-A ddPCR assay is able to determine MRD in MM subjects with high sensitivity, specificity, precision, and accuracy. Furthermore, the results of the CXCR3-A ddPCR assay correlate with the established NGF analysis of bone marrow aspirates of the subjects. This demonstrates that it was surprisingly possible to determine the MRD status of MM subjects by analyzing the expression of CXCR3-A on the surface of CD3+ T cells in the PB of the subjects.

The CXCR3-A ddPCR assay represents a revolutionary advancement in the detection of MRD in MM, offering significant advantages over current bone marrow-based and peripheral blood-based methods. By focusing on the functional expression of CXCR3-A in T cells, this assay bridges the gap between MRD detection and immune system dynamics, a critical but often overlooked factor in disease monitoring. Unlike bone marrow aspirates, which are invasive, painful, and impractical for frequent testing, the CXCR3-A ddPCR assay utilizes PB, enabling non-invasive and patient-friendly sampling that can be performed regularly. This allows for dynamic monitoring of disease status, facilitating earlier detection of relapses and timely therapeutic intervention.

Additionally, the assay's reliance on ddPCR ensures exceptional sensitivity and specificity, achieving a performance level comparable to the gold-standard methods, NGF and NGS. However, unlike these traditional approaches, the CXCR3-A ddPCR assay provides a functional perspective by measuring the expression of splice variant A of CXCR3, which reflects ongoing immune interactions driven by residual myeloma cells. This adds a novel layer of biological insight, as it highlights the immune system's response to disease progression, rather than focusing solely on the tumor burden. Moreover, the assay's simplicity, reproducibility, and low cost make it easily implementable in routine clinical laboratories, providing a widely accessible tool for MRD monitoring.

**Table 1 Summary of performance metrics of the CXCR3-A ddPCR assay**

| **Metric** | **Count** |
|---|---|
| True Positive (TP) | 13 |
| False Positive (FP) | 1* |
| True Negative (TN) | 17 |
| False Negative (FN) | 0 |
| Sensitivity (Recall) | 1 |
| Specificity | 0.9444 |
| Precision (PPV) | 0.9286 |
| Accuracy | 0.9677 |

| | |
|---|---|
| * The false positive case corresponds to a subject with an unusual extramedullary myeloma, where malignant plasma cells are located outside the bone marrow rather than within it. This highlights a potential limitation of bone marrow-based MRD detection using NGF, as the disease may not be captured in the sampled region. In contrast, our method, which relies on the immunological response and the expression of CXCR3 splice variants, may have successfully identified MRD in this rare presentation, underscoring its potential to detect residual disease even in cases with extramedullary involvement. | |

## Claims

1. An *in vitro* method for detecting multiple myeloma (MM) in a subject, wherein the method comprises:
a. obtaining a biological sample from the subject, and
b. determining the presence of the biomarker chemokine receptor CXCR3 (CXCR3) in the biological sample, wherein the presence of CXCR3 in the biological sample is indicative of MM in the subject.

2. The method according to claim 1, wherein the CXCR3 comprises or consists of at least one isoform of CXCR3, preferably CXCR3-A, CXCR3-B and/or CXCR3-alt, more preferably CXCR3-A.

3. The method according to any one of the preceding claims, wherein the biological sample is derived from the blood of the subject, preferably from the peripheral blood of the subject.

4. The method according to any one of the preceding claims, comprising detecting minimal residual disease (MRD) of MM in the subject.

5. The method according to any one of the preceding claims, wherein the determining in step b. comprises amplifying, using a polymerase chain reaction (PCR) assay, a target polynucleotide sequence corresponding to CXCR3 in the biological sample, thereby generating an amplified product.

6. The method according to claim 5, wherein the PCR assay is selected from a digital PCR (dPCR) and a real-time PCR (qPCR), preferably a dPCR, most preferably a droplet digital PCR (ddPCR).

7. The method according to claims 5 or 6, wherein the PCR is performed with primers and one or more DNA probes, preferably wherein at least one of the DNA probes is capable of binding to the target polynucleotide sequence, optionally wherein the primers and/or the one or more DNA probes comprise or consist of locked nucleic acids.

8. The method according to any one of the preceding claims, wherein between steps a. and b. step a.1 is performed, which comprises enriching an immune cell fraction in the biological sample, preferably enriching T cells in the biological sample, more preferably enriching CD3+ T cells in the biological sample.

9. Use of chemokine receptor CXCR3 (CXCR3) as a biomarker for diagnosing, prognosing, and/or treatment monitoring of multiple myeloma (MM) in a subject.

10. Use according to claim 9, comprising detecting minimal residual disease (MRD) of MM in the subject.

11. Use according to claim 9 or 10, wherein the CXCR3 comprises or consists of at least one isoform of CXCR3, more preferably CXCR3-A, CXCR3-B and/or CXCR3-alt, most preferably CXCR3-A.

12. Use according to any one of claims 9-11, wherein the CXCR3 is detected in a biological sample derived from the blood of the subject, preferably from the peripheral blood of the subject.

13. Use according to any one of claims 9-12, wherein the CXCR3 is detected by PCR.

14. Use of a method according to any one of claims 1-8 for diagnosing, prognosing, and/or treatment monitoring of multiple myeloma in a subject.

15. A kit for the detection of multiple myeloma in a subject, wherein the kit comprises:
- a first primer pair which is capable of amplifying a target polynucleotide sequence corresponding to the biomarker chemokine receptor CXCR3,
- a first DNA probe which is capable of binding to the target polynucleotide sequence,
- optionally a second primer pair which is capable of amplifying a reference polynucleotide sequence, and
- optionally a second DNA probe which is capable of binding to the reference polynucleotide sequence.
